# EUROPEAN PATENT APPLICATION

(11) **EP 4 119 099 A1**
(43) Date of publication of application: **18.01.2023**
(21) Application number: 21186249.5
(22) Date of filing: 16.07.2021
(51) Int. Cl.: A61F 2/30, A61F 2/44

(54) **EXPANDABLE AND ANGULARLY ADJUSTABLE SPINAL FUSION CAGES**

(71) Applicant: Blue Ocean Spine GmbH, 78532 Tuttlingen (DE)
(72) Inventor: SALVERMOSER, Markus, 78532 Möhringen (DE); RICHTER, Jacob, 78532 Tuttlingen (DE); EISEN, Guntmar, 78532 Tuttlingen (DE)
(74) Representative: Stolmár & Partner Patentanwälte PartG mbB

(57) **Abstract**

The present disclosure provides adjustable spinal devices, instruments for implanting the spinal devices, methods for adjusting the height and lordosis angles of the spinal devices and methods for implanting such devices. An adjustable spinal fusion device includes an upper plate component having an outer surface for placement against an endplate of a vertebral body and a lower plate component having an outer surface for placement against an endplate of a vertebral body. The device further includes a first translation member configured to move longitudinally relative to the upper and lower plates to adjust a distance between the upper and lower plates and a second translation member configured to move longitudinally relative to the upper and lower plates to adjust an angle between the upper and lower plates.

## Description

### FIELD

The present disclosure relates to implantable devices for promoting fusion of adjacent bony structures and, more particularly, to implantable spinal fusion cages that can adjust in height and angle to address lordosis within an intervertebral space.

### BACKGROUND

Spinal fusion cages can be used to treat a variety of spinal disorders, including degenerative disc disease. The cages provide a space for inserting a bone graft material between adjacent vertebrae, and to promote fusion between the bones at this spine segment.

A problem common to traditional fusion cages is their lack of ability to maintain or restore the normal anatomy of the fused spine segment. In order to insert the cage between the adjacent vertebra, at least a portion, if not all, of the intervertebral disc is removed to make room for the cage. The removal of the entire disc or disc portion disrupts the normal lordotic or kyphotic curvature of the spine. Traditional fusion cages do not attempt to correct this curvature, and over time as the vertebrae settle around the implanted cages, kyphotic deformity results.

It is therefore desirable to provide spinal fusion cages that have the ability to maintain and restore the normal anatomy of the fused spine segment.

### SUMMARY

The present disclosure provides adjustable spinal devices and instruments for implanting the spinal devices. The present disclosure further provides methods for adjusting the height and lordosis angles of the spinal devices and methods for implanting such devices.

In one embodiment, an adjustable spinal fusion device includes an upper plate component having an outer surface for placement against an endplate of a vertebral body and a lower plate component having an outer surface for placement against an endplate of a vertebral body. The device further includes a first translation member configured to move longitudinally relative to the upper and lower plates to adjust a distance between the upper and lower plates (i.e., the height of the implant); and a second translation member configured to move longitudinally relative to the upper and lower plates to adjust an angle between the upper and lower plates (i.e., the angle of lordosis of the implant).

It is to be understood that both the foregoing general description and the following detailed description are exemplary and explanatory only and are not restrictive of the disclosure. Additional features of the disclosure will be set forth in part in the description which follows or may be learned by practice of the disclosure.

### BRIEF DESCRIPTION OF THE DRAWINGS

The accompanying drawings, which are incorporated in and constitute a part of this specification, illustrate several embodiments of the disclosure and together with the description, serve to explain the principles of the disclosure.
FIGS. 1-12 illustrate various views of an implantable spinal device according to the present disclosure;
FIGS. 13-16 illustrate various views of an instrument for inserting the implantable spinal device of FIGS. 1-12 between adjacent vertebral bodies in a patient; and
FIGS. 17A-17C illustrate adjustment of the height and the angle of the implantable spinal device according to the present disclosure.

### DESCRIPTION OF THE EMBODIMENTS

This description and the accompanying drawings illustrate exemplary embodiments and should not be taken as limiting, with the claims defining the scope of the present disclosure, including equivalents. Various mechanical, compositional, structural, and operational changes may be made without departing from the scope of this description and the claims, including equivalents. In some instances, well-known structures and techniques have not been shown or described in detail so as not to obscure the disclosure. Like numbers in two or more figures represent the same or similar elements. Furthermore, elements and their associated aspects that are described in detail with reference to one embodiment may, whenever practical, be included in other embodiments in which they are not specifically shown or described. For example, if an element is described in detail with reference to one embodiment and is not described with reference to a second embodiment, the element may nevertheless be claimed as included in the second embodiment. Moreover, the depictions herein are for illustrative purposes only and do not necessarily reflect the actual shape, size, or dimensions of the system or illustrated components.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

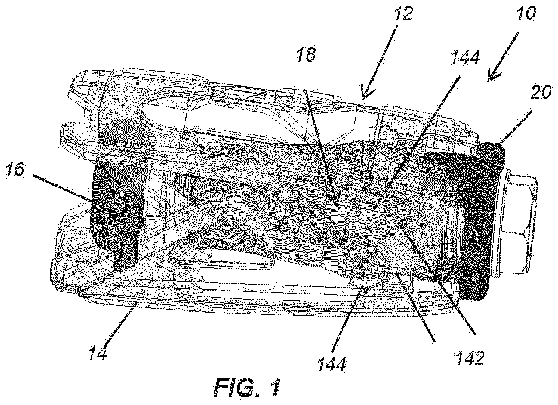

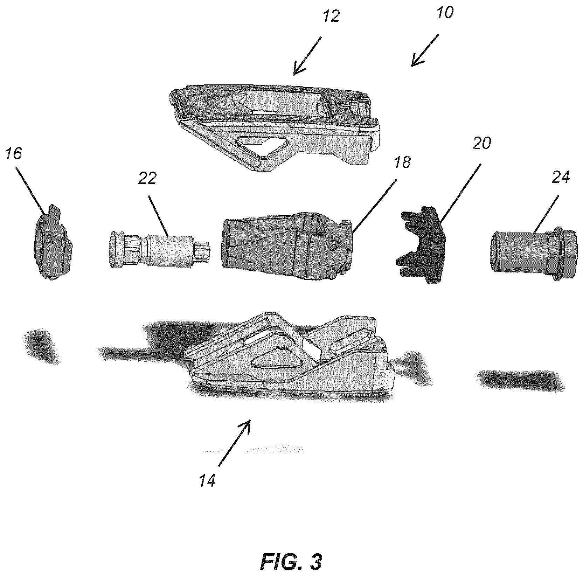

Referring now to FIGS. 1-3, a spinal implant 10 according to the present disclosure is configured for placement between two vertebral bodies, preferably from a posterior approach outside of the facet joint (transforaminal lumbar interbody fusion or TLIF). The implant 10 includes upper and lower endplates 12, 14, a distal translation member 16, a proximal translation member 18, and a proximal stabilization plate 20. The implant 10 further includes distal and proximal rotatable shaft actuators 22, 24 for longitudinally translating the distal and proximal translation members 16, 18, respectively, relative to the endplates 12, 14. Movement of the translation members 16, 18 in the longitudinal direction changes the height and angle of the endplates 12, 14, as discussed in more detail below.

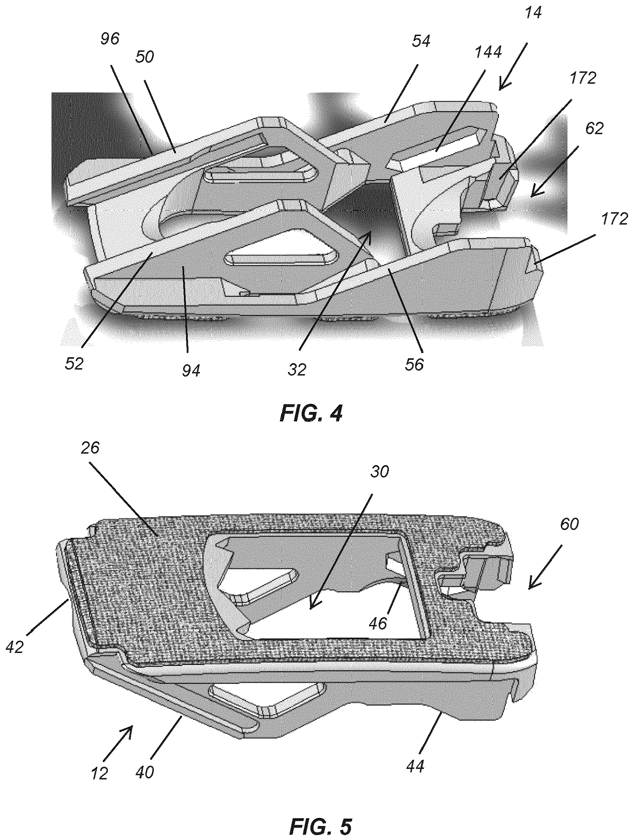

As shown in FIGS. 4 and 5, the upper and lower endplates 12, 14 each include an outer surface 26, 28 for contacting the surface of a vertebral body. The outer surfaces are preferably roughened with a surface treatment that facilitates attachment to the vertebral body. The surface treatment preferably creates a diamond structure (e.g., diamond 20-1.5), although other patterns may be used. The upper and lower endplates 12, 14 also include central openings 30, 32 that extend through the entire endplates 12, 14 and, in one embodiment, are substantially aligned with each other. Similarly, the proximal translation member 18 includes a central opening or bore 34 that, in one embodiment, may be substantially aligned with the endplate openings 30, 32. These openings create space for the addition of bone graft or other substances into the implant, as well as to allow for bony ingrowth through the implant 10.

The upper endplate 12 includes: (1) first and second distal sloped surfaces or ramps 40, 42 that are laterally spaced from each other near either side of the upper endplate and extend towards the lower plate 14 in the proximal direction; and (2) third and fourth proximal sloped surfaces or ramps 44, 46 that are laterally spaced from each other near either side of the endplate and also extend towards the lower endplate 14 in the proximal direction. The lower endplate 14 includes: (1) first and second distal sloped surfaces or ramps 50, 52 laterally spaced from each other extending upwards towards the upper endplate 12 in the proximal direction; and (2) third and fourth proximal sloped surfaces or ramps 54, 56 that are laterally spaced from each other near either side of the endplate and also extend upwards towards the upper endplate 12 in the proximal direction. These ramps interact with wedges on the translation members to provide height and angular adjustment of the implant.

The upper and lower endplates 12, 14 each further include a proximal cutout 60, 62 for receiving upper and lower portions of the stabilization plate 20, and vertical knobs or projections 64, 66 extending from the proximal translation member 18.

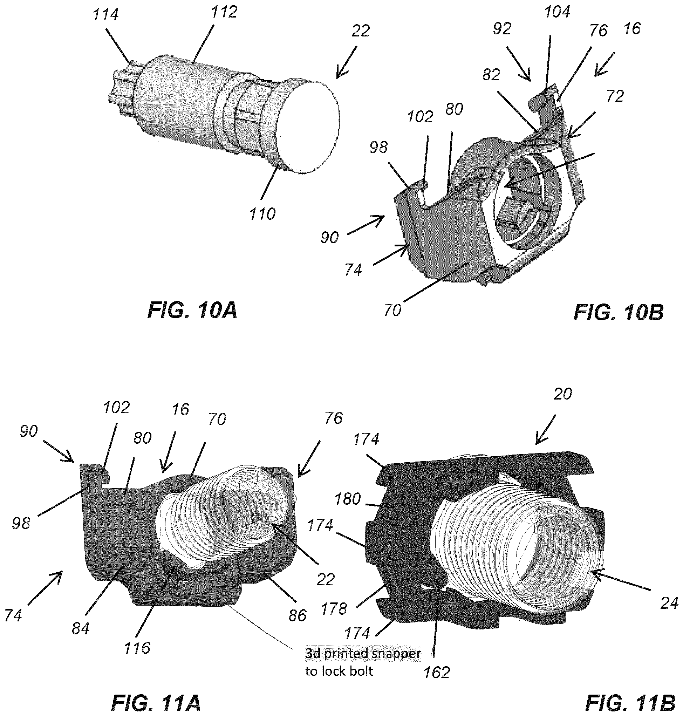

As shown in FIGS. 10B and 11A, the distal translation member 16 has a main body 70 positioned near the distal end of the endplates 12, 14 and forming a distal portion of the implant 10. The main body 70 includes a central bore 72 for receiving the distal shaft actuator 22 and first and second lateral portions 74, 76 extending laterally outward from the central bore 72. The lateral portions 74, 76 each further include: (1) first and second upper sloped surfaces or wedges 80, 82 laterally spaced from each other and extending downwards towards the lower endplate 14 in the proximal direction; and (2) first and second lower sloped surfaces or wedges 84, 86 laterally spaced from each other and extending upwards towards the upper endplate 12 in the proximal direction. The upper wedges 80, 82 are configured to contact and engage the upper endplate distal ramps 40, 42, and the lower wedges 84, 86 are configured to contact and engage the lower endplate distal ramps 50, 52 such that movement of distal translation member 16 in the proximal direction causes the proximal end of the endplates 12, 14 to move apart from each other. This movement may cause an adjustment in height or angle, as discussed below.

The lateral portions 74, 76 of the distal translation member 16 also include outer guide arms 90, 92 that engage with the outer surfaces 94, 96 of the distal ramps on the upper and lower endplates 12, 14 to stabilize the endplates during height and angle adjustment. Specifically, these outer guide arms 90, 92 comprise substantially vertical rails 98, 100 on either side of the distal translation member 16 that include projections 102, 104 designed to engage outer surface 94, 96 of the upper and lower distal endplate ramps and to ride along these ramps as the distal translation member 16 is moved in the longitudinal direction.

Referring now to FIG. 10A, the distal actuator shaft 22 includes a distal endplate or bolt 110, a threaded shaft 112 and a proximal mating feature, such as a shaped opening like, for example, a hexalobe 114, for mating with an instrument (discussed below). The distal actuator shaft 22 is secured to the distal translation member 16 with a flexible hinge or prong 116 that is biased inwards towards the longitudinal axis of the implant to secure the actuator shaft 22 to the distal translation member 16. The threaded shaft 112 also extends through a distal opening 124 in the proximal translation member 18 for mating with an instrument (discussed below).

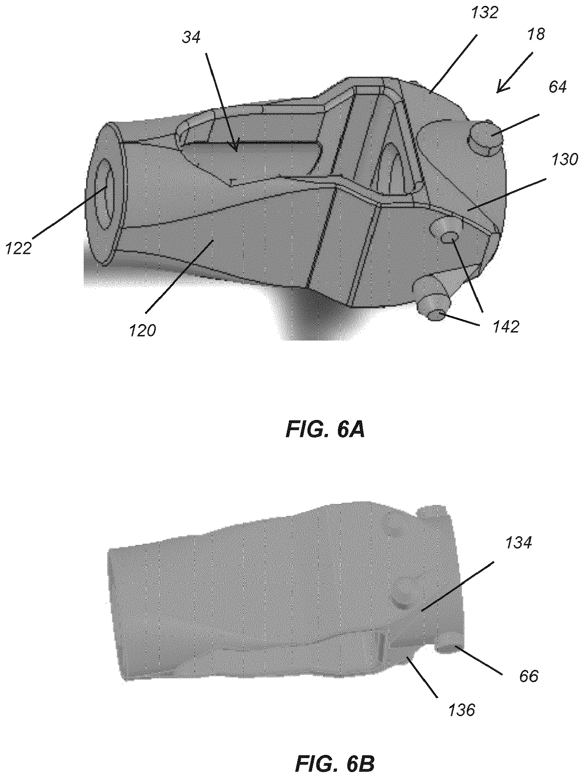

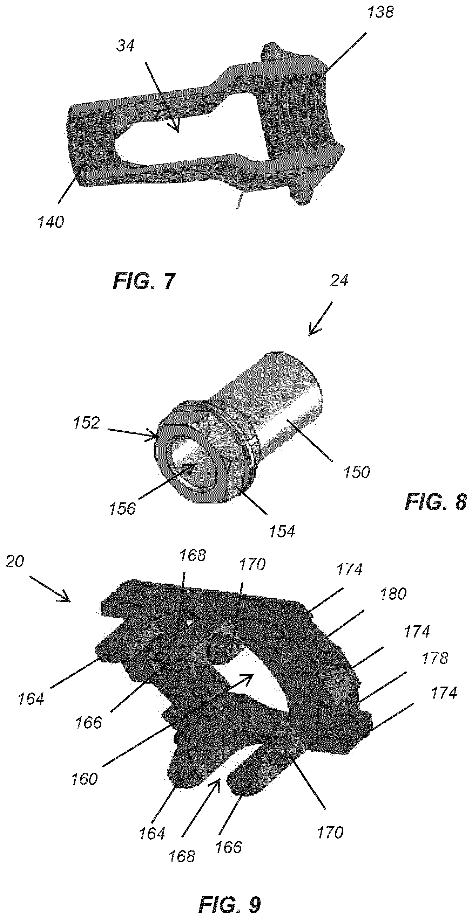

As shown in FIGS. 6A, 6B and 7, the proximal translation member 18 includes a substantially cylindrical main body 120 that extends through a portion of the implant between the endplates 12,14. The main body 120 has a central longitudinal bore 122 for receiving the proximal and distal actuator shafts 22, 24 and a central opening 34 extending from an upper surface through to the lower surface for receiving graft material and for allowing bony ingrowth therethrough. The proximal translation member 18 includes first and second upper sloped surfaces or wedges 130, 132 that are configured to contact and engage the first and second proximal ramps 44, 46 of the upper endplate 12, and first and second lower surfaces or wedges 134, 136 that are configured to contact and engage the first and second proximal ramps 54, 56 of the lower endplate 14. Longitudinal movement of proximal translation member 18 causes these wedges to move along the ramps of the endplates, thereby moving the proximal ends of the endplates either towards or away from each other.

The proximal translation member 18 further includes proximal internal threads 138 that rotatably mate with the threaded shaft 112 of the distal actuator shaft 22. Rotation of this threaded shaft 112 causes the distal translation member 16 to move longitudinally relative to the proximal translation member 18 and the end plates 12, 14 for angle adjustment, as discussed below. In addition, since the threaded shaft 112 of the distal actuator shaft 22 is coupled to the internal threads 138 of proximal translation member 18, longitudinal translation of the proximal translation member 18 relative to the endplates 12, 14 will also cause longitudinal translation of the distal translation member 16 in the same direction for height adjustment.

The proximal translation member 18 also includes distal internal threads 140 that are rotatably mated with a threaded shaft 150 of the proximal actuator 24. Rotation of the proximal actuator 24 causes proximal translation member 18 (and the distal translation member 16 therewith) to move longitudinally relative to the endplates 12, 14.

The proximal translation member 18 further includes one or more projections (i.e., conical pins 142) extending laterally away from the translation member. These conical pins 142 extend through angled slots 144 in the upper and lower endplates 12, 14 and serve to stabilize the endplates during height and angle adjustment. In particular, the proximal translation member 18 includes at least two conical pins 142 on each side, with one of the lateral pins on each side riding through slots 144 on the upper endplate and the other lateral pin riding through slots 144 on the lower endplate.

As shown in FIGS. 9 and 11B, the stabilization plate 20 includes a central opening 160 for receiving a portion of the proximal actuator shaft 24. The proximal actuator shaft 24 includes a proximal bolt 152 that is secured to the stabilization plate 20 with a hinge or prong 162 on the stabilization plate 20 that is biased inwards towards the longitudinal axis. The bolt 152 includes a mating feature 154 for engaging with a similar mating feature on an instrument (discussed below). The proximal actuator 24 further includes a central bore 156 for receiving an instrument to mate with the distal actuator 22, as discussed below.

The stabilization plate 20 is secured to the proximal actuator shaft 24 such that it remains fixed in place relative to the endplates 12, 14 as the translation members are moved in the longitudinal direction. The plate 20 includes two longitudinal projections 164, 166 on both of its upper and lower surfaces that extend towards the endplates to form a U-shaped projection with a central channel 168. The proximal translation member 18 includes upper and lower vertical projections 64, 66 configured to extend into these channels 168 and to move through the channels 168 as the proximal translation member 18 moves in the longitudinal direction. The U-shaped projections 164, 166 and channels 168 serve to stabilize the endplates and prevent seesaw or lateral movement of the proximal translation member. In addition, these U-shaped channels 164, 166 serve as a backstop to prevent excessive movement of the proximal translation member 18 in the proximal direction.

In addition, the stabilization plate further includes conical knobs 170 extending laterally from the longitudinal projections (two knobs on each side for a total of four conical knobs). These knobs are configured to slide within internal slots 172 on the upper and lower endplates to further stabilize the implant during height adjustment.

The stabilization plate further includes lateral projections 174 on either side of the plate that create two lateral grooves or channels 178, 180 for receiving projections on the insertion instrument (discussed below).

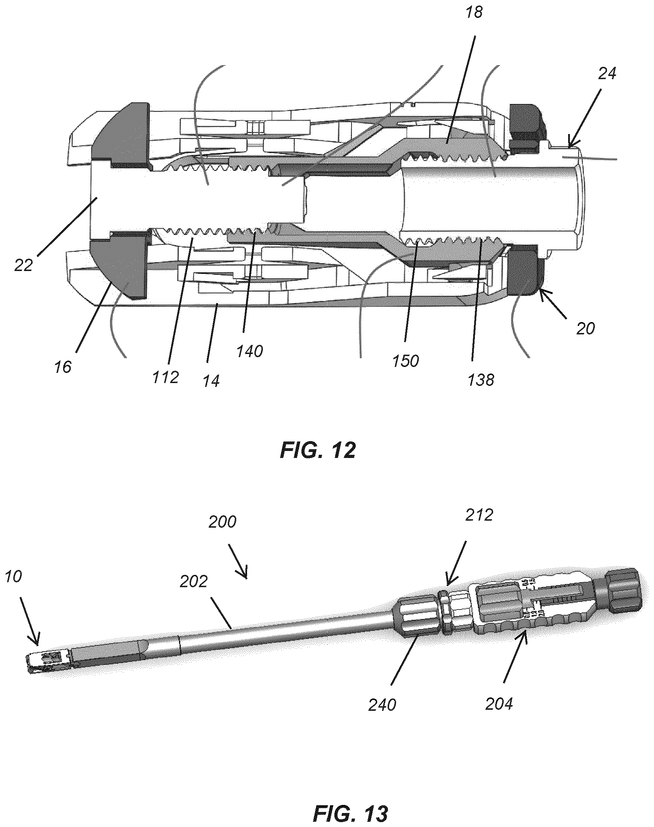

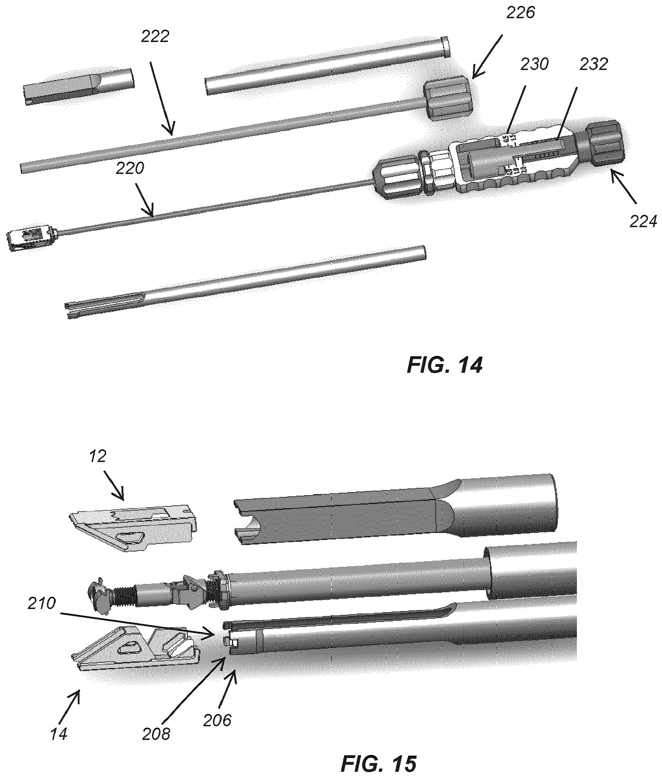

Referring now to FIGS. 13-16, the insertion instrument 200 comprises an elongated shaft 202 with a proximal handle 204 and a distal gripping element for 206 removable coupling to the implant 10. The distal gripping element 206 includes first and second gripping arms 208, 210 for coupling to the stabilization plate 20, i.e., to the lateral channels or grooves 178, 180 created by the lateral projections 174, 176 on the stabilization plate (a bayonet style connection). The distal gripping arms 208, 210 are coupled to an actuator 212 on the proximal handle 204 to move the arms 208, 210 in a substantially lateral direction relative to the longitudinal axis of the shaft 202. The arms 208, 210 can be moved together to hold the lower endplate and moved apart to release the endplate.

An inner rotatable shaft 220 extends from the handle 204 to the distal actuator 22 and an outer rotatable shaft 222 extends from the handle 204 to the proximal actuator 24. The inner and outer shafts 220, 222 are both attached to rotatable knobs 224, 226 on the proximal handle for rotating the shafts 220, 222 and thereby rotating the proximal and distal actuators 22, 24. The handle 204 includes markings or indicia 230, 232 to correlate rotation of the shafts with height and angle adjustment of the endplates. The inner shaft 220 has a female mating feature 234 that may be coupled to the male mating feature of the distal actuator (the hexalobe 114) and the outer shaft 222 has a female mating feature 236 that can be coupled to the bolt 152 on the proximal actuator 24.

The handle 204 further includes a third rotatable knob 240 coupled to the shaft 202 for rotating the shaft 202 and the endplate 10 therewith relative to the handle. This allows for rotation of the endplate without rotating the handle to facilitate ease of use during implantation.

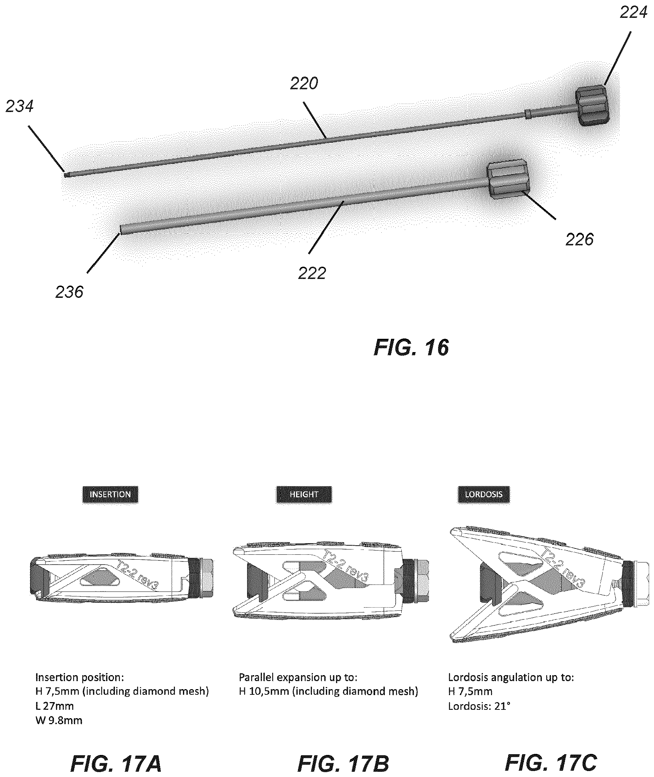

In use, the implant 10 may be advanced into an intervertebral space in a collapsed configuration (the resting state shown in FIG. 17A). To increase the height of the implant, the endplates 12, 14 are moved away from each other in a substantially parallel direction. To that end, the rotatable knob 226 on the handle 204 is rotated to thereby rotate the outer shaft 222 of the insertion instrument 200. This rotation causes the proximal actuator 24 to rotate, thereby causing its threaded shaft 150 to rotate within the proximal internal threads 138 of the proximal translation member 18. This translates both the proximal and distal translation members 16, 18 in the proximal direction. As the distal translation member 16 moves in the proximal direction, its upper wedges 80, 82 engage with the upper endplate distal ramps 40, 42 and its lower wedges 84, 86 with the lower endplate distal ramps 50, 52 such that the distal ends of the endplates move apart from each other. At the same time, the wedges 130, 132, 134, 136 of the proximal translation member 18 engage the proximal ramps 44, 46, 54, 56 of the endplates to move the proximal ends of the endplates away from each other. This causes the endplates to move away from each other in a substantially parallel direction (expanded state shown in FIG. 17B).

To adjust the angle of the endplates, the rotatable knob 224 on the handle 204 is rotated, causing the inner shaft 234 to rotate. This rotation is translated to the hexalobe 114 on the distal actuator 22. As the distal actuator 22 rotates, the distal translation member 16 is translated in a proximal direction relative to both the endplates 12, 14 and the proximal translation member 18. Since the proximal translation member 18 does not move, the proximal ends of the endplates remain fixed relative to each other. The upper and lower wedges of the distal translation member contact and engage the upper and lower distal ramps of the endplates, causing the distal ends of the endplates to move apart from each other, thereby adjusting the angle of the upper endplate relative to the lower endplate (angularly adjusted state shown in FIG. 17C).

The process of height and angle adjustment is reversible and stepless, i.e., a continuous adjustment without discrete steps. The height and angle may be adjusted independently of each other. For example, the above process can be reversed such that the inner shaft is first rotated to adjust the angle, and then the outer shaft is rotated to adjust height.

The entire implant is fabricated through additive manufacturing techniques, such as 3D printing. The implant is formed layer by layer in the longitudinal direction from the proximal end to the distal end. Upon completion of manufacturing, the upper and lower endplates are separated from each other. The endplates remain together during use by: (1) the knobs in the proximal translation member that slide through slots in the endplates; (2) the knobs in the stabilization plate that slide through slots in the endplates; (3) the side projections on the distal translation member that slide along the proximal ramps in the endplates; and (4) the upper and lower knobs in the proximal translation member that slide through the U-shaped channel in the stabilization plate.

In an exemplary embodiment, the implants are produced by Selective Laser Melting (SLM). For example, a substrate plate is fastened to an indexing table inside a chamber with a controlled atmosphere of inert gas (e.g., argon or nitrogen). Metal powder is applied flat to the substrate plate as a layer. The metal powder is preferably a titanium alloy, e.g. Ti-6AI-4V to enable biocompatibility. Each 2D slice of the cage is fused by selectively melting the metal powder via a laser. The laser has enough energy to fully melt or rather weld the metal particles to form solid metal. The substrate plate is lowered by the layer thickness (z-direction). New metal powder is applied and the process is repeated layer by layer until the part is complete. The completed part is removed from the substrate plate by cutting or breaking off.

Preferably, all components of the cage are printed nested within each other. Compared to separately 3D printing all components next to each other, a higher utilization rate can be achieved. This means that during 3D printing, a higher proportion which is melted and a lower proportion which stays as metal powder can be achieved. Thus, production time and costs can be reduced significantly.

After 3D printing, areas connecting single components of the cage are cut by electrical discharge machining (EDM) to enable their separate movement. Further, EDM can be used to realize smooth surfaces, e.g. to enable low-friction sliding of two components against each other. With EDM, the cage can also be removed from the substrate plate.

To lower production costs, several cages can be printed onto one substrate plate.

The implant may comprise one or more exhaust openings in the upper and lower endplates to allow for extraction of the metal powder remaining in the cage after 3D printing. Preferably, the exhaust opening is positioned on a lateral surface of the moving plate). It is also possible to position the exhaust opening on a horizontal surface of the cage, preferably on the base plate or on the moving plate. Preferably, the cage comprises multiple exhaust openings. Thus, more areas inside the cage are reachable and the metal powder can be extracted more efficiently. It is also possible to configure an external sliding mean, preferably a conical groove, in such a way that it can be additionally used as an exhaust opening. Therefore, the conical groove is deepened until a passage to the outside has been made.

Other embodiments will be apparent to those skilled in the art from consideration of the specification and practice of the embodiment disclosed herein. It is intended that the specification and examples be considered as exemplary only, with a true scope and spirit of the embodiment being indicated by the following claims.

## Claims

1. An adjustable spinal fusion device, comprising:
an upper plate component having an outer surface for placement against an endplate of a vertebral body;
a lower plate component having an outer surface for placement against an endplate of a vertebral body;
a first translation member configured to move longitudinally relative to the upper and lower plates to adjust a distance between the upper and lower plates; and
a second translation member configured to move longitudinally relative to the upper and lower plates to adjust an angle between the upper and lower plates.

2. The adjustable spinal fusion device of claim 1, further comprising an internal locking feature to prevent the upper plate from moving relative to the lower plate.

3. The adjustable spinal fusion device of claim 2, wherein the internal locking feature includes teeth for ratcheting.

4. The adjustable spinal fusion device of any preceding, further comprising a rotatable shaft coupled to the first translation member for moving the height translation member in the longitudinal direction.

5. The adjustable spinal fusion device of any preceding claim, further comprising a rotatable shaft coupled to the second translation member for moving the angle translation member in the longitudinal direction.

6. The adjustable spinal fusion device of any preceding claim, wherein the first translation member and/or the second translation member include/s a movable wedge.

7. The adjustable spinal fusion device of claim 6, wherein the upper and lower endplates each comprise a ramp for cooperating with the movable wedge/s.

8. The adjustable spinal fusion device of any preceding claim, further being configured for use in the lumbar spine.
